(19) **Europäisches Patentamt / European Patent Office / Office européen des brevets**

(11) **EP 4 429 558 B1**

(12) # EUROPEAN PATENT SPECIFICATION

(45) Date of publication and mention
of the grant of the patent:
**18.02.2026   Bulletin 2026/08**

(21) Application number: **21810982.5**

(22) Date of filing: **11.11.2021**

(51) International Patent Classification (IPC):
**A61B 8/00** *(2006.01)*      **A61B 8/08** *(2006.01)*

(52) Cooperative Patent Classification (CPC):
**A61B 8/5261; A61B 8/4245; A61B 8/523**

(86) International application number:
**PCT/EP2021/081378**

(87) International publication number:
**WO 2023/083452 (19.05.2023 Gazette 2023/20)**

(54) **ULTRASOUND TO 3D REGISTRATION USING A-MODE SIGNALS**

ULTRASCHALL-AUF-3D-REGISTRIERUNG UNTER VERWENDUNG VON A-MODUS-SIGNALEN

ENREGISTREMENT D'ULTRASONS VERS 3D À L'AIDE DE SIGNAUX EN MODE A

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**18.09.2024   Bulletin 2024/38**

(73) Proprietor: **Brainlab SE
81829 München (DE)**

(72) Inventors:
 • **WEI, Wei
  81829 Munich (DE)**
 • **MENA, Daniel
  81829 Munich (DE)**

(74) Representative: **SSM Sandmair
Patentanwälte Rechtsanwalt
Partnerschaft mbB
Joseph-Wild-Straße 20
81829 München (DE)**

(56) References cited:
**US-A- 6 106 464**

 • **HEGER S ET AL: "User-interactive registration of
bone with a-mode ultrasound", IEEE
ENGINEERING IN MEDICINE AND BIOLOGY
MAGAZINE, IEEE SERVICE CENTER,
PISACATAWAY, NJ, US, vol. 24, no. 2, 2 March
2005 (2005-03-02), pages 85 - 95, XP011129133,
ISSN: 0739-5175, DOI: 10.1109/
MEMB.2005.1411353**
 • **AL-HASSAN ALY ET AL: "On Ultrasound Imaging
for Guided Screw Insertion in Spinal Fusion
Surgery", ULTRASOUND IN MEDICINE AND
BIOLOGY, NEW YORK, NY, US, vol. 37, no. 4, 14
January 2011 (2011-01-14), pages 651 - 664,
XP028367538, ISSN: 0301-5629, [retrieved on
20110126], DOI: 10.1016/
J.ULTRASMEDBIO.2011.01.011**

**Description**

**FIELD OF THE INVENTION**

The present invention relates to a computer-implemented method for calculating a registration of a 3D image dataset of a patient taken at a first point in time with ultrasound data of the patient taken at a second point in time later than the first point in time, a corresponding computer program, a computer-readable storage medium storing such a program and a computer executing the program.

**TECHNICAL BACKGROUND**

The present invention has the object of matching ultrasound data captured using an ultrasound transducer with existing 3D image data, for example CT or MRT image data.

The present invention can be used in connection with a system for surgical navigation such as Curve®, Kick® or Buzz®, each products of Brainlab AG.

Aspects of the present invention, examples and exemplary steps and their embodiments are disclosed in the following. Different exemplary features of the invention can be combined in accordance with the invention wherever technically expedient and feasible.

Document US 6,106,464 A discloses an A-mode ultrasound transducer tracked in three-dimensions by an optical position tracking system as the transducer is scanned over the skin to generate measurements of bone surface distance from the transducer.

A processor correlates the ultrasound data with position and orientation data to generate a three-dimensional physical space model of the bone surface which is registered with an image space model of the bone surface generated from a tomographic image to produce an alignment of the two models.

HEGER S ET AL: "User-interactive registration of bone with a-mode ultrasound", IEEE ENGINEERING IN MEDICINE AND BIOLOGY MAGAZINE, IEEE SERVICE CENTER, PISACATAWAY, NJ, US, vol. 24, no. 2, 1 March 2005 (2005-03-01), pages 85-95, discloses the use of an A-mode ultrasound probe tracked by a three-dimensional (3-D) localizer system for noninvasive transcutaneous palpation and registration of bone surface points.

AL-HASSAN ALY ET AL: "On Ultrasound Imaging for Guided Screw Insertion in Spinal Fusion Surgery", ULTRASOUND IN MEDICINE AND BIOLOGY, NEW YORK, NY, US, vol. 37, no. 4, 14 January 2011 (2011-01-14), pages 651-664, describes an image guidance technique based on generating B-mode images from within a small bore hole in a pedicle's trabecular bone.

**EXEMPLARY SHORT DESCRIPTION OF THE INVENTION**

In the following, a short description of the specific features of the present invention is given which shall not be understood to limit the invention only to the features or a combination of the features described in this section.

In many medical applications, it is crucial to match or register two different image data with each other, for example live image data with previously obtained 3D image data. The present invention relates to registration of a 3D image dataset with (live) ultrasound data. But rather than reconstructing an image from the ultrasound data and registering the reconstructed image with the 3D image dataset, the approach of the present invention matches the ultrasound A mode signals output by an ultrasound transducer with simulated ultrasound A mode signals generated from the 3D image dataset.

When the 3D image dataset is registered with the ultrasound data, ultrasound images generated from the ultrasound data can for example be supplemented with data taken from the 3D image dataset.

**GENERAL DESCRIPTION OF THE INVENTION**

In this section, a description of the general features of the present invention is given for example by referring to possible embodiments of the invention.

An object of the present invention is calculating a registration of a 3D image dataset of a patient taken at a first point in time with ultrasound data of the patient taken at a second point in time later than the first point in time. The 3D image dataset represents a 3D image of at least a part of the anatomical structure of the patient and represents, for example, a CT image or an MRT image.

The ultrasound data is captured using an ultrasound transducer comprising a plurality of transducer elements. Each transducer element emits an ultrasound wave, also referred to as ultrasound signal, and transforms the returning ultrasound wave into an electrical signal having an amplitude which varies over time depending on how the emitted ultrasound wave is refracted or reflected by the anatomical structure of the patient. This electrical signal over time is also referred to as an ultrasound A mode signal or radio frequency (RF) data. Each transducer element outputs an ultrasound A mode signal. The ultrasound A mode signals output by the ultrasound transducer are referred to as measured ultrasound A mode signals and form the ultrasound data. The transducer elements are typically arranged in a particular pattern, such as a line or two-dimensional array, typically in an equidistant arrangement.

In general, the invention reaches the aforementioned object by providing, in a first aspect, a computer-implemented data processing method of calculating a registration of a 3D image dataset of a patient taken at a first point in time with ultrasound data of the patient taken at a second point in time later than the first point in time. The method comprises executing, on at least one processor of at least one computer (for example at least one computer being part of a navigation system), the following exemplary steps.

In a (for example first) exemplary step, the ultrasound data is acquired. The ultrasound data comprises a measured set of measured ultrasound A mode signals. The measured set typically comprises one measured ultrasound A mode signal for each transducer element.

Each transducer element has an ultrasound emitter which emits the ultrasound wave and an ultrasound detector for receiving the returning sound wave and generating the output signal. The ultrasound transmitter and the ultrasound detector can be the same element, such as a transceiver using a piezoelectric element, for example. The measured ultrasound A mode signals can be the raw output signals of the transducer elements or can be filtered output signals, for example using a lowpass filter or a bandpass filter in order to remove noise from the raw output signals.

In a (for example second) exemplary step, the registration is calculated for which the similarity between the measured set and a simulated set of simulated ultrasound A mode signals generated from the 3D image dataset is maximized.

The 3D image dataset has an associated reference system, for example a Cartesian coordinate system having three mutually perpendicular axes. An ultrasound image reconstructed from the ultrasound data also has an associated reference system, in particular a Cartesian coordinate system having three mutually perpendicular axes. The reference system of the ultrasound image is typically defined relative to the ultrasound transducer, such that it can also be referred to as the reference system of the ultrasound transducer.

A simulated ultrasound A mode signal is synthesized or simulated from the 3D image dataset and an assumed position of a virtual transducer element relative to the 3D image dataset, in particular within the reference system of the 3D image dataset. The simulated ultrasound A mode signal represents the theoretical output signal of the virtual transducer element when it is at the virtual position relative to the 3D image dataset, transmits an ultrasound wave to the 3D image dataset and outputs the returning ultrasound wave as a virtual output signal having an amplitude over time.

The simulated set of simulated ultrasound A mode signals is generated for a virtual ultrasound transducer having the same geometry as the real ultrasound transducer used for capturing the ultrasound data and using the same parameters, like the same transmission sequence. There is a one-to-one correspondence of each transducer element of the real ultrasound transducer with one transducer element of the virtual ultrasound transducer. The generation of the simulated ultrasound A mode signals utilizes the known specifications of the ultrasound transducer, such as the geometry, the frequency, the number of transducer elements and the properties of the emitted ultrasound wave.

The result of the registration of the 3D image dataset with the ultrasound data is for example the position of the reference system of the 3D image dataset in the reference system of the ultrasound transducer or vice versa.

A simulated set of simulated ultrasound A mode signals is calculated for a particular virtual position of the virtual ultrasound transducer in the reference system of the 3D image dataset and is compared to the measured set of measured ultrasound A mode signals. The comparison results in a single similarity value belonging to the virtual position of the virtual ultrasound transducer. The virtual relative position which results in the largest similarity value is the one which maximizes the similarity and represents the registration between the 3D image dataset and the ultrasound data or, in other words, between the 3D image dataset and the ultrasound transducer.

The 3D image dataset and the ultrasound data represent the same part of the patient. So if the virtual position of the virtual ultrasound transducer when generating the simulated set of simulated ultrasound A mode signals corresponds to the relative position of the real ultrasound transducer relative to the patient, each measured ultrasound A mode signal should be identical or at least highly similar to the corresponding simulated ultrasound A mode signal.

The disadvantage of the traditional approach of reconstructing a 3D image from the ultrasound data and registering the 3D image dataset with the reconstructed 3D ultrasound image is that the reconstructed 3D ultrasound image typically has a low image quality due to noise or other influences. In addition, reconstructing a 3D ultrasound image from the ultrasound data requires lots of computational resources.

With the approach of the present invention, a simulated set of simulated ultrasound A mode signals can be generated from the 3D image dataset in advance, such that it is not necessary to generate it in real time, and only the simulated ultrasound A mode signals have to be compared to the measured ultrasound A mode signals, which is possible in real time.

Simulating ultrasound A mode signals is known to the skilled person, for example from:

1. J.A. Jensen: Field: A Program for Simulating Ultrasound Systems, Paper presented at the 10th Nordic-Baltic Conference on Biomedical Imaging Published in Medical & Biological Engineering & Computing, pp. 351-353, Volume 34, Supplement 1, Part 1, 1996.

2. J.A. Jensen and N. B. Svendsen: Calculation of pressure fields from arbitrarily shaped, apodized, and excited ultrasound transducers, IEEE Trans. Ultrason., Ferroelec., Freq. Contr., 39, pp. 262-267, 1992.

3. B. E. Treeby and B. T. Cox, "k-Wave: MATLAB toolbox for the simulation and reconstruction of photoacoustic wavefields," J. Biomed. Opt., vol. 15, no. 2, p. 021314, 2010.

4. Garcia, Damien. "SIMUS: an open-source simulator for ultrasound imaging. Part I: theory & examples." arXiv preprint arXiv:2102.02738 (2021).

5. Cigier, Amanda, François Varray, and Damien Garcia. "SIMUS: an open-source simulator for ultrasound imaging. Part II: comparison with three popular simulators." arXiv preprint arXiv:2103.05521 (2021).

In one embodiment, as explained above, the simulated set of simulated ultrasound A mode signals is calculated for a virtual position of an ultrasound transducer used for measuring the measured ultrasound A mode signals relative to the 3D image dataset. The ultrasound transducer has a plurality of ultrasound detectors and each ultrasound detector measures one of the measured ultrasound A mode signals in the measured set.

Since there is a one-to-one correspondence between the virtual ultrasound transducer and the real ultrasound transducer, it is known which measured ultrasound A mode signal of the measured set corresponds to which simulated ultrasound A mode signal in the simulated set. It is thus known which simulated and which measured ultrasound A mode signal have to be compared when calculating the similarity measure value.

According to the invention, calculating the registration involves calculating a plurality of simulated sets of ultrasound A mode signals for different relative positions between the 3D image dataset and the ultrasound data. Those different relative positions are for example different virtual positions of the virtual ultrasound transducer relative to the 3D image dataset. The embodiment further involves using the simulated set out of the plurality of simulated sets for which the similarity is the largest. This simulated set having the largest similarity can be selected, searched or predicted using any suitable algorithm.

In a brute force approach, each of the simulated sets is compared to the measured set, and each comparison results in a similarity value. The relative position of the simulated set which leads to the largest similarity value represents the registration between the 3D image dataset and the ultrasound data. However, any suitable optimizer can be employed to find the global maximum of the similarity value without comparing each of the simulated sets to the measured set.

The similarity between the measured set and the simulated set is calculated using any suitable method, like normalized correlation, mutual information, gradient correlation or gradient information.

Normalized correlation is explained in Dickey, Fred M., and Louis A. Romero. "Normalized correlation for pattern recognition." Optics letters 16.15 (1991): 1186-1188.

Mutual information is explained in Gierlichs, Benedikt, et al. "Mutual information analysis." International Workshop on Cryptographic Hardware and Embedded Systems. Springer, Berlin, Heidelberg, 2008.

Gradient correlation is explained in Becke, Axel D. "Density-functional thermochemistry. II. The effect of the Perdew-Wang generalized-gradient correlation correction." The Journal of chemical physics 97.12 (1992): 9173-9177.

Gradient information is explained in Sun, Changming. "Symmetry detection using gradient information." Pattern Recognition Letters 16.9 (1995): 987-996.

In one implementation, a signal similarity value is calculated for each pair of a measured ultrasound A mode signal and the corresponding simulated ultrasound A mode signal and the overall similarity value is calculated from the set of signal similarity values, for example by accumulating or averaging the signal similarity values.

In one embodiment, the similarity is maximized using an optimizer like covariance matrix adaptation evolution strategy. CMAES is decribed in Loshchilov, Ilya. "CMA-ES with restarts for solving CEC 2013 benchmark problems." 2013 IEEE Congress on Evolutionary Computation. Ieee, 2013.

In one implementation, the similarity between a measured ultrasound A mode signal and a simulated ultrasound A mode signal is calculated by the following equation:

$$Similarity(\overrightarrow{S(x,y,z,\alpha,\beta,\gamma)_{ij}},\overrightarrow{M_{ij}})$$

$$= \frac{\sum_{d=0}^{d\leq k}(\overrightarrow{S(x,y,z,\alpha,\beta,\gamma)_{ij}}[d] - Mean_s) * (\overrightarrow{M_{ij}}[d] - Mean_m)}{\sqrt{\sum_{d=0}^{d\leq k}(\overrightarrow{S(x,y,z,\alpha,\beta,\gamma)_{ij}}[d] - Mean_s)^2} * \sqrt{\sum_{d=0}^{d\leq k}(\overrightarrow{M_{ij}}[d] - Mean_m)^2}}$$

with

$$Mean_s = \frac{\sum_{d=0}^{d\leq k}(\overrightarrow{S(x,y,z,\alpha,\beta,\gamma)_{ij}}[d])}{k}$$

and

$$Mean_m = \frac{\sum_{d=0}^{d\leq k}(\overrightarrow{M_{ij}}[d])}{k}.$$

In this equation, $\overrightarrow{S(x,y,z,\alpha,\beta,\gamma)_{ij}}$ is the simulated ultrasound A mode signal of transducer element (i, j) of a virtual ultrasound transducer having m x n transducer elements, wherein $0 \leq i < m$ and $0 \leq j < n$, for a particular set of parameters x, y, z, $\alpha$, $\beta$, $\gamma$. Those parameters define the virtual position of the virtual ultrasound probe relative to the 3D image dataset in three translational dimensions x, y and z and three rotational dimensions $\alpha$, $\beta$ and $\gamma$. $\overrightarrow{M_{ij}}$ is the measured ultrasound A mode signal of transducer element (i, j) of the real ultrasound transducer. The simulated and measured ultrasound A mode signals are sampled signals having k samples each.

Maximizing the similarity can then be performed using the following equation:

$$Argmax_{(x,y,z,\alpha,\beta,\gamma)}(\sum_{i=0}^{m-1}\sum_{j=0}^{n-1}Similarity(\overrightarrow{S(x,y,z,\alpha,\beta,\gamma)_{ij}},\overrightarrow{M_{ij}})).$$

This means that the parameters x, y, z, $\alpha$, $\beta$ and $\gamma$ are found for which the similarity between $\overrightarrow{S(x,y,z,\alpha,\beta,\gamma)}$ and $\overrightarrow{M}$ is maximized. This overall similarity is calculated by summing up all similarities between the m * n pairs of a simulated ultrasound A mode signal and a corresponding measured ultrasound A mode signal.

Maximizing the similarity can involve finding the $\overrightarrow{S(x,y,z,\alpha,\beta,\gamma)}$ which is most similar to the $\overrightarrow{M}$ among an existing plurality of sets of simulated ultrasound A mode signals, numerically calculating this $\overrightarrow{S(x,y,z,\alpha,\beta,\gamma)}$ or a combination thereof.

According to the invention, the method further comprises the step of generating an ultrasound model including tissue properties from the 3D image dataset. Generating the simulated set of simulated ultrasound A mode signals then involves simulating the propagation of an ultrasound signal, or ultrasound wave, from the virtual ultrasound transducer through the ultrasound model back to the virtual ultrasound transducer.

In one implementation, the ultrasound model is further based on an atlas of the patient which represents acoustic tissue properties. The atlas is for example matched to the 3D image dataset and the ultrasound model is derived from the matched atlas. The atlas can be a generic atlas or a patient-specific atlas.

In another implementation, the 3D image dataset is analysed to estimate Hounsfield units or intensity values for the different tissue types derived from the 3D image dataset. The ultrasound model is then based on the estimated tissue types.

The generation of ultrasound models from 3D image data is known, for example from:

- Dillenseger, Jean-Louis & Laguitton, Soizic & Delabrousse, Eric. (2009). Fast simulation of ultrasound images from a CT volume. Computers in biology and medicine. 39. 180-6. 10.1016/j.compbiomed.2008.12.009.

- Shams R., Hartley R., Navab N. (2008) Real-Time Simulation of Medical Ultrasound from CT Images. In: Metaxas D., Axel L., Fichtinger G., Székely G. (eds) Medical Image Computing and Computer-Assisted Intervention - MICCAI 2008. MICCAI 2008. Lecture Notes in Computer Science, vol 5242. Springer, Berlin, Heidelberg. https://doi.org/10.1007/978-3-540-85990-1_88

- Wein W., Khamene A., Clevert DA., Kutter O., Navab N. (2007) Simulation and Fully Automatic Multimodal Registration of Medical Ultrasound. In: Ayache N., Ourselin S., Maeder

In one embodiment, the similarity is optimized in a multi-dimensional search space. This multi-dimensional search space has up to three translational dimensions and up to three rotational dimensions. The search space defines the position of the reference system of the ultrasound data or the ultrasound transducer in the reference system of the 3D image dataset or vice versa.

In order to optimize the calculation of the registration, the multi-dimensional search space can be limited, resulting in a limited search space. This in particular defines the boundaries for potential virtual positions of the virtual ultrasound transducer relative to the 3D image dataset.

In one embodiment, calculating the registration involves the step of forming a thinned-out measured subset of the measured set. This means that only some of the measured ultrasound A mode signals are comprised in the thinned-out measured subset.

It further involves forming a thinned-out simulated subset from the simulated set, the thinned-out simulated subset corresponding to the thinned-out measured subset. This means that the thinned-out simulated subset comprises those simulated ultrasound A mode signals for which the thinned-out measured subset comprises a measured ultrasound A mode signal.

It further comprises the step of calculating an intermediate registration for which the similarity between the thinned-out measured set and the thinned-out simulated set is maximized. In other words, the intermediate registration is not based on the full measured set and the full simulated set, but on sparse versions of those sets in order to decrease the computational complexity.

It further involves the step of defining a limited search space around the intermediate registration. Due to the sparse versions of the measured set and simulated set, the intermediate registration is assumed to be approximately correct, but not the optimum. It is assumed that the optimum registration lies within the limited search space.

In this embodiment, calculating the registration then further involves the step of calculating the registration for which the similarity between the measured set and the simulated set is maximized within the limited search space. In this last step, the full measured set and the full simulated set are used, but only for finding a registration within the limited search space.

The full simulated sets are only needed within the limited search space, but not in the preceding steps of setting the limited search space. This can reduce the computational burden when calculating simulated ultrasound A mode signals.

In one implementation, calculating the registration involves the step of tracking the position of the ultrasound transducer used for measuring the measured set relative to the patient and setting a limited search space based on the position of the ultrasound transducer. Since the 3D image dataset represents a particular part of the patient, the position of the ultrasound transducer relative to the patient also allows an approximation of the registration of the 3D image dataset with the ultrasound data. The exact registration for which the similarity between the measured set and a simulated set is maximized is then calculated within the limited search space.

In one implementation, this embodiment further comprises the steps of registering an atlas of the patient with the 3D image dataset and determining the position of the 3D image dataset relative to the patient based on the registered atlas. The registered atlas of the patient is a model of the patient and the registration of the atlas with the 3D image dataset finds the position of the 3D image dataset relative to the atlas, and thus relative to the patient. The step of setting the limited search space is then also based on the position of the 3D image dataset relative to the patient. In this implementation, an assumption of the position of the 3D image dataset relative to the patient is replaced by a determined relative position based on the matched atlas.

Rather than tracking the position of the ultrasound transducer, the position of the ultrasound transducer relative to the patient can be derived from the ultrasound data captured using the ultrasound transducer. The position of the ultrasound transducer relative to the patient can for example be determined based on typical patterns in the ultrasound data, and thus the measured ultrasound A mode signals, or a B mode ultrasound image reconstructed from the measured ultrasound A mode signals, for a particular relative position. Alternatively, artificial intelligence could be trained to approximate the position of the ultrasound transducer relative to the patient based on the measured ultrasound data.

In one embodiment, calculating the registration involves the step of registering an atlas of the patient with the 3D image dataset and determining the surface of the patient from the registered atlas. The atlas defines the structure of the patient, and thus the location of the skin surface. When the atlas is registered with the 3D image dataset, the skin surface relative to the 3D image dataset is known. Since it can be assumed that the ultrasound transducer rests on the skin of the patient when capturing the ultrasound data, the limited search space can be set based on the surface of the patient, for example two locations within one millimeter, five millimeters, one centimeter or two centimeters in a direction orthogonal to the skin surface. This is because the ultrasound transducer typically pushes the skin slightly inwards when it is held against the patient.

Besides limiting the search space, the computational complexity can additionally or alternatively be reduced by optimizing the calculation of the similarity.

In one embodiment, the simulated set is smaller than the measured set, wherein the simulated ultrasound A signals to be calculated are set based on marks in the 3D image dataset or based on labels in an atlas matched to the 3D image dataset. The labels can for example indicate that the ultrasound signal emitted by the ultrasound transducer hits a structure such that the returning ultrasound wave measured by the detector in the ultrasound transducer is not meaningful. In this case, it can be determined that some of the measured ultrasound A mode signals do not meaningfully contribute to a similarity. So corresponding simulated ultrasound A mode signals do not have to be calculated, or, if already present, selected to be compared with measured ultrasound A mode signals, thus reducing the computational complexity. The similarity is then calculated from those measured ultrasound A mode signals in the measured set which correspond to the simulated (or selected) ultrasound A mode signals in the simulated set. This does not only reduce the amount of processing required for simulating the simulated ultrasound A mode signals, but also reduces the complexity when calculating the similarity.

In one embodiment, calculating the registration involves the steps of grouping simulated ultrasound A mode signals from the simulated set into at least one simulated group and calculating a simulated group signal for each of the at least one simulated group. In other words, the plurality of simulated ultrasound A mode signals of the simulated group is reduced to a single simulated group signal.

Calculating the registration further involves the steps of grouping measured ultrasound A mode signals from the measured set into at least one measured group corresponding to the at least one simulated group and calculating a measured group signal for each of the measured groups. Like for the simulated ultrasound A mode signals, those steps combine the measured ultrasound A mode signals of a measured group into a single measured group signal for this measured group. The simulated ultrasound A mode signal is grouped into a particular simulated group corresponding to the measured ultrasound A mode signals grouped into a corresponding measured group in a one-to-one relationship such that the transducer element of the real ultrasound transducer which captures a measured ultrasound A mode signal corresponds to the same transducer element of the virtual ultrasound transducer for which the simulated ultrasound A mode signal is calculated.

The similarity in the step of calculating the registration is then the similarity between the simulated group signals and the measured group signals.

This embodiment has the advantage that the number of comparisons between simulated ultrasound A mode signals and measured ultrasound A mode signals can be significantly reduced.

The atlas mentioned above can also be used for supplementing the 3D image dataset, for example when generating the ultrasound model. For example if the 3D image dataset represents an internal part of the patient, but does not include a skin surface, data for generating the ultrasound model based on estimated tissue types might not be possible. In this case, areas of the patient necessary for generating the ultrasound model but not comprised in the 3D image dataset, can be taken or derived from the atlas.

The acoustic tissue properties included in the ultrasound model for example include the acoustical impedance, speckle and speed of sound.

In a second aspect, the invention is directed to a computer program comprising instructions which, when the program is executed by at least one computer, causes the at least one computer to carry out method according to the first aspect. The invention may alternatively or additionally relate to a (physical, for example electrical, for example technically generated) signal wave, for example a digital signal wave, such as an electromagnetic carrier wave carrying information which represents the program, for example the aforementioned program, which for example comprises code means which are adapted to perform any or all of the steps of the method according to the first aspect. The signal wave is in one example a data carrier signal carrying the aforementioned computer program. A computer program stored on a disc is a data file, and when the file is read out and transmitted it becomes a data stream for example in the form of a (physical, for example electrical, for example technically generated) signal. The signal can be implemented as the signal wave, for example as the electromagnetic carrier wave which is described herein. For example, the signal, for example the signal wave is constituted to be transmitted via a computer network, for example LAN, WLAN, WAN, mobile network, for example the internet. For example, the signal, for example the signal wave, is constituted to be transmitted by optic or acoustic data transmission. The invention according to the second aspect therefore may alternatively or additionally relate to a data stream representative of the aforementioned program, i.e. comprising the program.

In a third aspect, the invention is directed to a computer-readable storage medium on which the program according to the second aspect is stored. The program storage medium is for example non-transitory.

In a fourth aspect, the invention is directed to at least one computer (for example, a computer), comprising at least one processor (for example, a processor), wherein the program according to the second aspect is executed by the processor, or wherein the at least one computer comprises the computer-readable storage medium according to the third aspect.

For example, the invention does not involve or in particular comprise or encompass an invasive step which would represent a substantial physical interference with the body requiring professional medical expertise to be carried out and entailing a substantial health risk even when carried out with the required professional care and expertise.

## DEFINITIONS

In this section, definitions for specific terminology used in this disclosure are offered which also form part of the present disclosure.

### Computer implemented method

The method in accordance with the invention is for example a computer implemented method. For example, all the steps or merely some of the steps (i.e. less than the total number of steps) of the method in accordance with the invention can be executed by a computer (for example, at least one computer). An embodiment of the computer implemented method is a use of the computer for performing a data processing method. An embodiment of the computer implemented method is a method concerning the operation of the computer such that the computer is operated to perform one, more or all steps of the method.

The computer for example comprises at least one processor and for example at least one memory in order to (technically) process the data, for example electronically and/or optically. The processor being for example made of a substance or composition which is a semiconductor, for example at least partly n- and/or p-doped semiconductor, for example at least one of II-, III-, IV-, V-, VI-semiconductor material, for example (doped) silicon and/or gallium arsenide. The calculating or determining steps described are for example performed by a computer. Determining steps or calculating steps are for example steps of determining data within the framework of the technical method, for example within the framework of a program. A computer is for example any kind of data processing device, for example electronic data processing device. A computer can be a device which is generally thought of as such, for example desktop PCs, notebooks, netbooks, etc., but can also be any programmable apparatus, such as for example a mobile phone or an embedded processor. A computer can for example comprise a system (network) of "sub-computers", wherein each sub-computer represents a computer in its own right. The term "computer" includes a cloud computer, for example a cloud server. The term computer includes a server resource. The term "cloud computer" includes a cloud computer system which for example comprises a system of at least one cloud computer and for example a plurality of operatively interconnected cloud computers such as a server farm. Such a cloud computer is preferably connected to a wide area network such as the world wide web (WWW) and located in a so-called cloud of computers which are all connected to the world wide web. Such an infrastructure is used for "cloud computing", which describes computation, software, data access and storage services which do not require the end user to know the physical location and/or configuration of the computer delivering a specific service. For example, the term "cloud" is used in this respect as a metaphor for the Internet (world wide web). For example, the cloud provides computing infrastructure as a service (IaaS). The cloud computer can function as a virtual host for an operating system and/or data processing application which is used to execute the method of the invention. The cloud computer is for example an elastic compute cloud (EC2) as provided by Amazon Web Services™. A computer for example comprises interfaces in order to receive or output data and/or perform an analogue-to-digital conversion. The data are for example data which represent physical properties and/or which are generated from technical signals. The technical signals are for example generated by means of (technical) detection devices (such as for example devices for detecting marker devices) and/or (technical) analytical devices (such as for example devices for performing (medical) imaging methods), wherein the technical signals are for example electrical or optical signals. The technical signals for example represent the data received or outputted by the computer. The computer is preferably operatively coupled to a display device which allows information outputted by the computer to be displayed, for example to a user. One example of a display device is a virtual reality device or an augmented reality device (also referred to as virtual reality glasses or augmented reality glasses) which can be used as "goggles" for navigating. A specific example of such augmented reality glasses is Google Glass (a trademark of Google, Inc.). An augmented reality device or a virtual reality device can be used both to input information into the computer by user interaction and to display information outputted by the computer. Another example of a display device would be a standard computer monitor comprising for example a liquid crystal display operatively coupled to the computer for receiving display control data from the computer for generating signals used to display image information content on the display device. A specific embodiment of such a computer monitor is a digital lightbox. An example of such a digital lightbox is Buzz®, a product of Brainlab AG. The monitor may also be the monitor of a portable, for example handheld, device such as a smart phone or personal digital assistant or digital media player.

The invention also relates to a computer program comprising instructions which, when on the program is executed by a computer, cause the computer to carry out the method or methods, for example, the steps of the method or methods, described herein and/or to a computer-readable storage medium (for example, a non-transitory computer-readable storage medium) on which the program is stored and/or to a computer comprising said program storage medium and/or to a (physical, for example electrical, for example technically generated) signal wave, for example a digital signal wave, such as an electromagnetic carrier wave carrying information which represents the program, for example the aforementioned program, which for example comprises code means which are adapted to perform any or all of the method steps described herein. The signal wave is in one example a data carrier signal carrying the aforementioned computer program. The invention also relates to a computer comprising at least one processor and/or the aforementioned computer-readable storage medium and for example a memory, wherein the program is executed by the processor.

Within the framework of the invention, computer program elements can be embodied by hardware and/or software (this includes firmware, resident software, micro-code, etc.). Within the framework of the invention, computer program elements can take the form of a computer program product which can be embodied by a computer-usable, for example computer-readable data storage medium comprising computer-usable, for example computer-readable program instructions, "code" or a "computer program" embodied in said data storage medium for use on or in connection with the instruction-executing system. Such a system can be a computer; a computer can be a data processing device comprising means for executing the computer program elements and/or the program in accordance with the invention, for example a data processing device comprising a digital processor (central processing unit or CPU) which executes the computer program elements, and optionally a volatile memory (for example a random access memory or RAM) for storing data used for and/or produced by executing the computer program elements. Within the framework of the present invention, a computer-usable, for example computer-readable data storage medium can be any data storage medium which can include, store, communicate, propagate or transport the program for use on or in connection with the instruction-executing system, apparatus or device. The computer-usable, for example computer-readable data storage medium can for example be, but is not limited to, an electronic, magnetic, optical, electromagnetic, infrared or semiconductor system, apparatus or device or a medium of propagation such as for example the Internet. The computer-usable or computer-readable data storage medium could even for example be paper or another suitable medium onto which the program is printed, since the program could be electronically captured, for example by optically scanning the paper or other suitable medium, and then compiled, interpreted or otherwise processed in a suitable manner. The data storage medium is preferably a non-volatile data storage medium. The computer program product and any software and/or hardware described here form the various means for performing the functions of the invention in the example embodiments. The computer and/or data processing device can for example include a guidance information device which includes means for outputting guidance information. The guidance information can be outputted, for example to a user, visually by a visual indicating means (for example, a monitor and/or a lamp) and/or acoustically by an acoustic indicating means (for example, a loudspeaker and/or a digital speech output device) and/or tactilely by a tactile indicating means (for example, a vibrating element or a vibration element incorporated into an instrument). For the purpose of this document, a computer is a technical computer which for example comprises technical, for example tangible components, for example mechanical and/or electronic components. Any device mentioned as such in this document is a technical and for example tangible device.

**Acquiring data**

The expression "acquiring data" for example encompasses (within the framework of a computer implemented method) the scenario in which the data are determined by the computer implemented method or program. Determining data for example encompasses measuring physical quantities and transforming the measured values into data, for example digital data, and/or computing (and e.g. outputting) the data by means of a computer and for example within the framework of the method in accordance with the invention. A step of "determining" as described herein for example comprises or consists of issuing a command to perform the determination described herein. For example, the step comprises or consists of issuing a command to cause a computer, for example a remote computer, for example a remote server, for example in the cloud, to perform the determination. Alternatively or additionally, a step of "determination" as described herein for example comprises or consists of receiving the data resulting from the determination described herein, for example receiving the resulting data from the remote computer, for example from that remote computer which has been caused to perform the determination. The meaning of "acquiring data" also for example encompasses the scenario in which the data are received or retrieved by (e.g. input to) the computer implemented method or program, for example from another program, a previous method step or a data storage medium, for example for further processing by the computer implemented method or program. Generation of the data to be acquired may but need not be part of the method in accordance with the invention. The expression "acquiring data" can therefore also for example mean waiting to receive data and/or receiving the data. The received data can for example be inputted via an interface. The expression "acquiring data" can also mean that the computer implemented method or program performs steps in order to (actively) receive or retrieve the data from a data source, for instance a data storage medium (such as for example a ROM, RAM, database, hard drive, etc.), or via the interface (for instance, from another computer or a network). The data acquired by the disclosed method or device, respectively, may be acquired from a database located in a data storage device which is operably to a computer for data transfer between the database and the computer, for example from the database to the computer. The computer acquires the data for use as an input for steps of determining data. The determined data can be output again to the same or another database to be stored for later use. The database or database used for implementing the disclosed method can be located on network data storage device or a network server (for example, a cloud data storage device or a cloud server) or a local data storage device (such as a mass storage device operably connected to at least one computer executing the disclosed method). The data can be made "ready for use" by performing an additional step before the acquiring step. In accordance with this additional step, the data are generated in order to be acquired. The data are for example detected or captured (for example by an analytical device). Alternatively or additionally, the data are inputted in accordance with the additional step, for instance via interfaces. The data generated can for example be inputted (for instance into the computer). In accordance with the additional step (which precedes the acquiring step), the data can also be provided by performing the additional step of storing the data in a data storage medium (such as for example a ROM, RAM, CD and/or hard drive), such that they are ready for use within the framework of the method or program in accordance with the invention. The step of "acquiring data" can therefore also involve commanding a device to obtain and/or provide the data to be acquired. In particular, the acquiring step does not involve an invasive step which would represent a substantial physical interference with the body, requiring professional medical expertise to be carried out and entailing a substantial health risk even when carried out with the required professional care and expertise. In particular, the step of acquiring data, for example determining data, does not involve a surgical step and in particular does not involve a step of treating a human or animal body using surgery or therapy. In order to distinguish the different data used by the present method, the data are denoted (i.e. referred to) as "XY data" and the like and are defined in terms of the information which they describe, which is then preferably referred to as "XY information" and the like.

**Image registration**

Image registration is the process of transforming different sets of data into one coordinate system and getting them aligned to each other. The data can be multiple photographs and/or data from different sensors, different times or different viewpoints. It is used in computer vision, medical imaging and in compiling and analysing images and data from satellites. Registration is necessary in order to be able to compare or integrate the data obtained from these different measurements.

**Marker**

It is the function of a marker to be detected by a marker detection device (for example, a camera or an ultrasound receiver or analytical devices such as CT or MRI devices) in such a way that its spatial position (i.e. its spatial location and/or alignment) can be ascertained. The detection device is for example part of a navigation system. The markers can be active markers. An active marker can for example emit electromagnetic radiation and/or waves which can be in the infrared, visible and/or ultraviolet spectral range. A marker can also however be passive, i.e. can for example reflect electromagnetic radiation in the infrared, visible and/or ultraviolet spectral range or can block x-ray radiation. To this end, the marker can be provided with a surface which has corresponding reflective properties or can be made of metal in order to block the x-ray radiation. It is also possible for a marker to reflect and/or emit electromagnetic radiation and/or waves in the radio frequency range or at ultrasound wavelengths. A marker preferably has a spherical and/or spheroid shape and can therefore be referred to as a marker sphere; markers can however also exhibit a cornered, for example cubic, shape.

**Marker device**

A marker device can for example be a reference star or a pointer or a single marker or a plurality of (individual) markers which are then preferably in a predetermined spatial relationship. A marker device comprises one, two, three or more markers, wherein two or more such markers are in a predetermined spatial relationship. This predetermined spatial relationship is for example known to a navigation system and is for example stored in a computer of the navigation system.

In another embodiment, a marker device comprises an optical pattern, for example on a two-dimensional surface. The optical pattern might comprise a plurality of geometric shapes like circles, rectangles and/or triangles. The optical pattern can be identified in an image captured by a camera, and the position of the marker device relative to the camera can be determined from the size of the pattern in the image, the orientation of the pattern in the image and the distortion of the pattern in the image. This allows determining the relative position in up to three rotational dimensions and up to three translational dimensions from a single two-dimensional image.

The position of a marker device can be ascertained, for example by a medical navigation system. If the marker device is attached to an object, such as a bone or a medical instrument, the position of the object can be determined from the position of the marker device and the relative position between the marker device and the object. Determining this relative position is also referred to as registering the marker device and the object. The marker device or the object can be tracked, which means that the position of the marker device or the object is ascertained twice or more over time.

**Tracking system**

A tracking system, such as a surgical tracking system, is understood to mean a system which can comprise: at least one marker device; a transmitter which emits electromagnetic waves and/or radiation and/or ultrasound waves; a receiver which receives electromagnetic waves and/or radiation and/or ultrasound waves; and an electronic data processing device which is connected to the receiver and/or the transmitter, wherein the data processing device (for example, a computer) for example comprises a processor (CPU) and a working memory, wherein the data processing device processes navigation data forwarded to it by the receiver and can determine the position of the at least one marker device in a particular reference system, such as the reference system of the tracking system.

**Atlas / Atlas segmentation**

Preferably, atlas data is acquired which describes (for example defines, more particularly represents and/or is) a general three-dimensional shape of an anatomical body part. The atlas data therefore represents an atlas of the anatomical body part. An atlas typically consists of a plurality of generic models of objects, wherein the generic models of the objects together form a complex structure. For example, the atlas constitutes a statistical model of a patient's body (for example, a part of the body) which has been generated from anatomic information gathered from a plurality of human bodies, for example from medical image data containing images of such human bodies. In principle, the atlas data therefore represents the result of a statistical analysis of such medical image data for a plurality of human bodies. This result can be output as an image - the atlas data therefore contains or is comparable to medical image data. Such a comparison can be carried out for example by applying an image fusion algorithm which conducts an image fusion between the atlas data and the medical image data. The result of the comparison can be a measure of similarity between the atlas data and the medical image data. The atlas data comprises image information (for example, positional image information) which can be matched (for example by applying an elastic or rigid image fusion algorithm) for example to image information (for example, positional image information) contained in medical image data so as to for example compare the atlas data to the medical image data in order to determine the position of anatomical structures in the medical image data which correspond to anatomical structures defined by the atlas data.

The human bodies, the anatomy of which serves as an input for generating the atlas data, advantageously share a common feature such as at least one of gender, age, ethnicity, body measurements (e.g. size and/or mass) and pathologic state. The anatomic information describes for example the anatomy of the human bodies and is extracted for example from medical image information about the human bodies. The atlas of a femur, for example, can comprise the head, the neck, the body, the greater trochanter, the lesser trochanter and the lower extremity as objects which together make up the complete structure. The atlas of a brain, for example, can comprise the telencephalon, the cerebellum, the diencephalon, the pons, the mesencephalon and the medulla as the objects which together make up the complex structure. One application of such an atlas is in the segmentation of medical images, in which the atlas is matched to medical image data, and the image data are compared with the matched atlas in order to assign a point (a pixel or voxel) of the image data to an object of the matched atlas, thereby segmenting the image data into objects.

For example, the atlas data includes information of the anatomical body part. This information is for example at least one of patient-specific, non-patient-specific, indication-specific or non-indication-specific. The atlas data therefore describes for example at least one of a patient-specific, non-patient-specific, indication-specific or non-indication-specific atlas. For example, the atlas data includes movement information indicating a degree of freedom of movement of the anatomical body part with respect to a given reference (e.g. another anatomical body part). For example, the atlas is a multimodal atlas which defines atlas information for a plurality of (i.e. at least two) imaging modalities and contains a mapping between the atlas information in different imaging modalities (for example, a mapping between all of the modalities) so that the atlas can be used for transforming medical image information from its image depiction in a first imaging modality into its image depiction in a second imaging modality which is different from the first imaging modality or to compare (for example, match or register) images of different imaging modality with one another.

**Imaging methods**

In the field of medicine, imaging methods (also called imaging modalities and/or medical imaging modalities) are used to generate image data (for example, two-dimensional or three-dimensional image data) of anatomical structures (such as soft tissues, bones, organs, etc.) of the human body. The term "medical imaging methods" is understood to mean (advantageously apparatus-based) imaging methods (for example so-called medical imaging modalities and/or radiological imaging methods) such as for instance computed tomography (CT) and cone beam computed tomography (CBCT, such as volumetric CBCT), x-ray tomography, magnetic resonance tomography (MRT or MRI), conventional x-ray, sonography and/or ultrasound examinations, and positron emission tomography. For example, the medical imaging methods are performed by the analytical devices. Examples for medical imaging modalities applied by medical imaging methods are: X-ray radiography, magnetic resonance imaging, medical ultrasonography or ultrasound, endoscopy, elastography, tactile imaging, thermography, medical photography and nuclear medicine functional imaging techniques as positron emission tomography (PET) and Single-photon emission computed tomography (SPECT), as mentioned by Wikipedia.

The image data thus generated is also termed "medical imaging data". Analytical devices for example are used to generate the image data in apparatus-based imaging methods. The imaging methods are for example used for medical diagnostics, to analyse the anatomical body in order to generate images which are described by the image data. The imaging methods are also for example used to detect pathological changes in the human body. However, some of the changes in the anatomical structure, such as the pathological changes in the structures (tissue), may not be detectable and for example may not be visible in the images generated by the imaging methods. A tumour represents an example of a change in an anatomical structure. If the tumour grows, it may then be said to represent an expanded anatomical structure. This expanded anatomical structure may not be detectable; for example, only a part of the expanded anatomical structure may be detectable. Primary/high-grade brain tumours are for example usually visible on MRI scans when contrast agents are used to infiltrate the tumour. MRI scans represent an example of an imaging method. In the case of MRI scans of such brain tumours, the signal enhancement in the MRI images (due to the contrast agents infiltrating the tumour) is considered to represent the solid tumour mass. Thus, the tumour is detectable and for example discernible in the image generated by the imaging method. In addition to these tumours, referred to as "enhancing" tumours, it is thought that approximately 10% of brain tumours are not discernible on a scan and are for example not visible to a user looking at the images generated by the imaging method.

## BRIEF DESCRIPTION OF THE DRAWINGS

In the following, the invention is described with reference to the appended figures which give background explanations and represent specific embodiments of the invention. The scope of the invention is however not limited to the specific features disclosed in the context of the figures, wherein

Figure 1    shows a navigated ultrasound transducer,
Figure 2    shows a measured ultrasound A mode signal,
Figure 3    shows an array of transducer elements of the ultrasound transducer,
Figure 4    shows a system for implementing the invention,
Figure 5    shows a flow diagram of the present invention, and
Figure 6    shows a schematic overview of the concept of the present invention.

## DESCRIPTION OF EMBODIMENTS

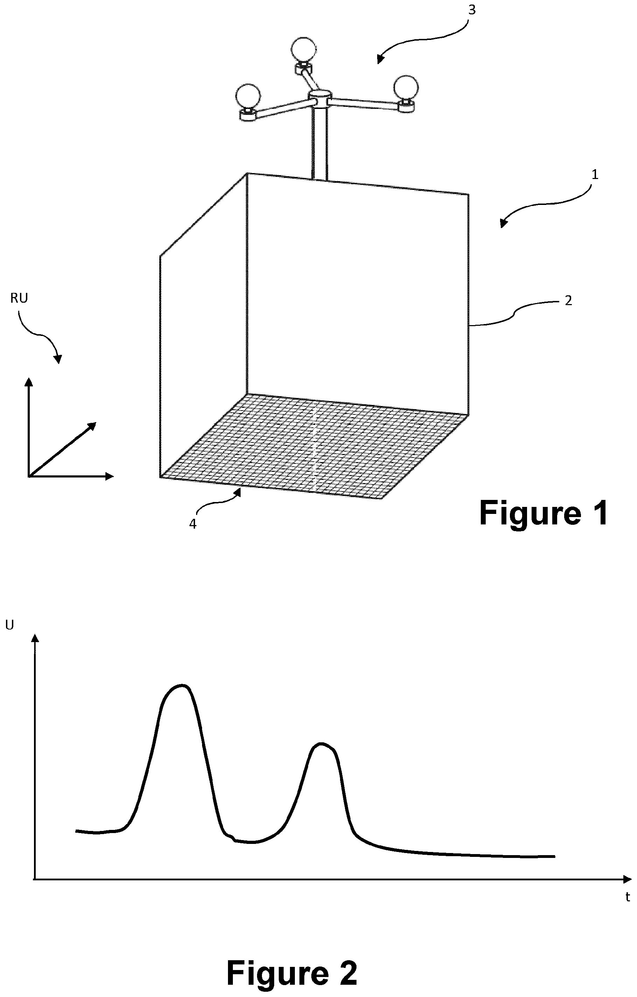

Figure 1 shows a perspective view of an exemplary ultrasound transducer 1. The ultrasound transducer 1 has a body 2 to which a marker device 3 is attached. In the present example, the marker device 3 has three marker spheres in known positions relative to the body 2 of the ultrasound transducer 1. By determining the positions of the marker spheres using a medical tracking system, the position of the ultrasound transducer 1 can be determined, for example relative to the medical tracking system.

One surface of the body 2 of the ultrasound transducer 1 comprises a plurality of transducer elements 4. Each transducer element 4 acts as an emitter to emit an ultrasound wave, which is also referred to as ultrasound signal, and as a detector element to detect incident ultrasound energy. In Figure 1, the array of transducer elements is arranged on a flat surface, but could for example also be arranged on a curved surface of the body 2 of the ultrasound transducer 1.

Each transducer element 4 outputs a measured ultrasound A mode signal. An envelope of an exemplary measured ultrasound A mode signal is shown in Figure 2. The measured ultrasound A mode signal represents an amplitude, in the present example a voltage, over time. The voltage represents the strength of the incident ultrasound signal at the corresponding transducer element 4. The example of Figure 2 shows two pronounced echoes caused by reflections of the emitted ultrasound signal at transitions from one tissue type to another tissue type along the propagation path of the ultrasound signal.

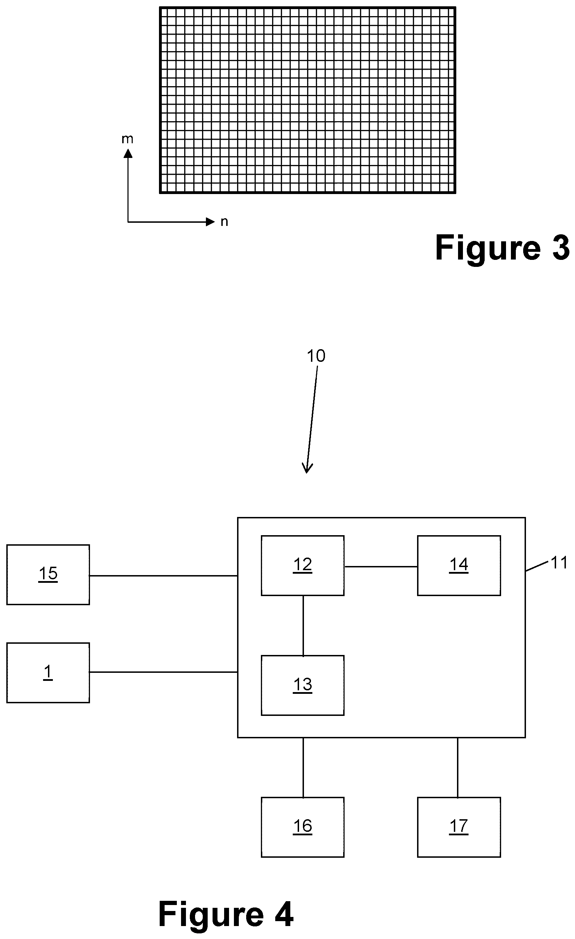

Figure 3 shows a plan view of the array of transducer elements 4. The position of a particular transducer element 4 on the surface is identified by indices m and n. m and n can be integers starting at (0,0) and counting the number of transducer elements in two orthogonal directions.

The ultrasound signal is typically emitted perpendicular to the surface of the corresponding transducer element 4, is reflected, absorbed, scattered or otherwise modified when propagating through the tissue of the patient and returns to the detector of the corresponding transducer element 4 where the incident ultrasound energy is translated into the measured ultrasound A mode signal. The measured ultrasound A mode signal therefore depends on the tissue structure of the patient. It further depends on the properties of the transducer element 4, such as the waveform of the emitted ultrasound signal, in particular its frequency, and the properties of the ultrasound detector.

The ultrasound transducer 1 of Figure 1 generates a measured set including a plurality of measured ultrasound A mode signals. From this measured set, a 2D, 3D or 4D ultrasound image can be reconstructed.

The ultrasound transducer 1 has an associated reference system RU, in the present example consisting of three mutually orthogonal axes. A reconstructed ultrasound image is defined in this reference system RU.

Figure 4 shows an exemplary system 10 for implementing the present invention. The system 10 comprises a computer 11 connected to an ultrasound transducer 1, a tracking system 15, an input device 16 and an output device 17. The input device 16 can be any suitable input device, such as a keyboard, a mouse, a trackball or a touchpad. The output device 17 can be any suitable output device, such as a monitor, a touch sensitive display, a projector or a wearable display.

The computer 11 comprises a central processing unit 12, an interface 13 and a memory 14. The central processing unit 12 executes a program which implements the present invention and is for example stored in the memory 14. The memory 14 can further store processed data or data to be processed, such as a 3D image dataset to be registered with ultrasound data generated by the ultrasound transducer 1.

The interface 13 is configured to connect the computer 11 to external devices, such as the ultrasound transducer 1, the tracking system 15, the input device 16 and the output device 17.

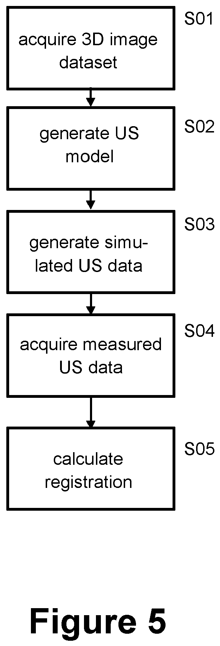

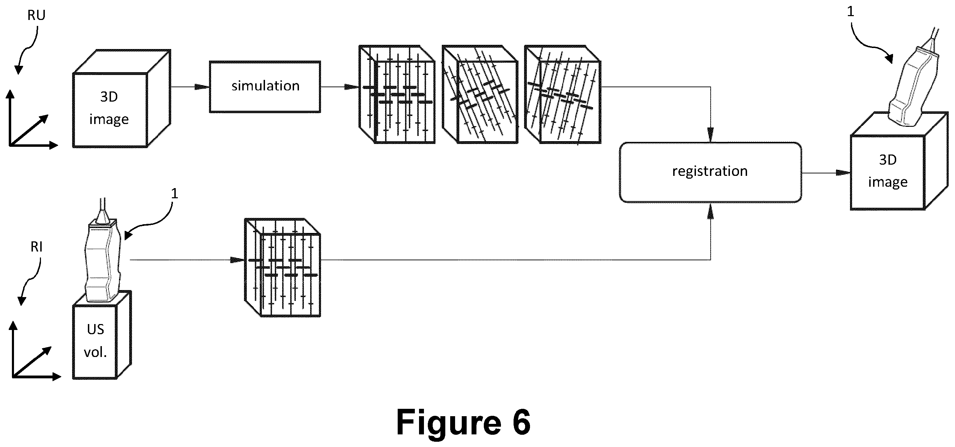

Figure 5 shows an exemplary flow diagram of the method according to the present invention and Figure 6 visualizes the concept of the present invention.

At step S01, a 3D image dataset is acquired by the computer 11 and stored in the memory 14. The 3D image dataset can be acquired from an external apparatus, such as a 3D imaging device like a CT or MRT device, or from an external storage, which could be provided locally or accessed over a network. The 3D image dataset represents a 3D image of at least a part of the patient and is for example given as a three-dimensional array of voxels each having assigned a particular value. The 3D image dataset has an associated image reference system RI.

As explained above, the purpose of the present invention is finding a registration between the 3D image dataset and the ultrasound data generated by the ultrasound transducer 1. This registration is for example represented by the position of the reference system RI of the 3D image dataset in the reference system RU of the ultrasound transducer 1, and thus of the ultrasound data, for which an ultrasound image constructed from the ultrasound data best matches the 3D image represented by the 3D image dataset.

Step S02 involves generating an ultrasound model based on the 3D image dataset. The ultrasound model can be calculated from the 3D image dataset alone, for example by translating the voxel values of the 3D image into acoustic properties. Generation of the ultrasound model could additionally involve use of an atlas comprising acoustic tissue properties. The atlas is then matched to the 3D image dataset and the data of the matched atlas is used to generate the ultrasound model.

The ultrasound model is based on the 3D image dataset and is thus defined in the same reference system RI.

Based on the generated ultrasound model, step S03 involves generating simulated ultrasound data. In particular, a plurality of simulated sets each comprising a plurality of simulated ultrasound A mode signals is generated from the ultrasound model, and thus indirectly from the 3D image dataset, for different virtual positions of the virtual ultrasound transducer relative to the ultrasound model.

The three cubes to the right of the simulator in Figure 6 show paths of ultrasound signals of three exemplary simulated sets of simulated ultrasound A mode signals. In this example, each simulated set comprises a 3x3 array of simulated ultrasound A mode signals. As can be seen from those three cubes, the three simulated sets are calculated for different positions of the virtual ultrasound transducer relative to the 3D image dataset.

The virtual position of the virtual ultrasound transducer relative to a 3D image dataset / ultrasound model is represented by the position of the reference system RI of the 3D image dataset in the reference system RU of the virtual ultrasound transducer.

Step S04 involves acquiring measured ultrasound data comprising a measured set of measured ultrasound A mode signals. This is shown in the lower part of Figure 6, where the ultrasound transducer 1 probes an ultrasound volume of the patient which at least partly overlaps with the volume of the patient represented by the 3D image dataset.

The virtual ultrasound transducer used for calculating the simulated sets of simulated ultrasound A mode signals has the same properties as the real ultrasound transducer 1, and in particular the same number and arrangement of transducer elements 4. The measured ultrasound data is represented in the lower part of Figure 6 by an exemplary cube showing 3x3 measured ultrasound A mode signals.

Step S05 involves calculating the registration between the 3D image dataset and the measured ultrasound data as represented by the box "registration" in Figure 6.

In the simplest implementation, this step involves selecting the simulated set of simulated ultrasound A mode signals having the largest similarity with the measured set of measured ultrasound A mode signals.

Calculating the similarity for example involves a pair-wise comparison of the simulated ultrasound A mode signals with the corresponding measured ultrasound A mode signals and to combine all results of the pair-wise comparisons into a single similarity value.

The right most part of Figure 6 shows the result of the registration in which the position of the 3D image dataset relative to the ultrasound transducer 1 is determined. With this relative position, the ultrasound image reconstructed from the ultrasound data coincides with the 3D image represented by the 3D image dataset. When the ultrasound image, or a part thereof, is displayed, for example on the output device 17, additional information taken from the registered 3D image dataset can be added.

The step of calculating the registration can trigger the generation of new simulated sets for new relative positions of the virtual ultrasound transducer, for example if the similarity does not fulfil predetermined criteria, such as reaching a particular threshold.

The simulated sets of simulated ultrasound A mode signals can be calculated and stored in advance, thus reducing the computational complexity when calculating the registration. Modern storage means allow storage of an extremely large number of simulated sets. However, in this case, a large number of simulated sets has to be compared to the measured set if a brute force approach is applied. It is thus advantageous to limit the number of simulated sets to be compared to the measured set. This can for example be achieved by tracking the ultrasound transducer 1 using the tracking system 15.

The approximate position of the 3D image dataset relative to the patient is typically known, for example due to a known position of the patient relative to the imaging device when the 3D image dataset is recorded. By tracking the ultrasound transducer 1 using the tracking system 15, the approximate position of the ultrasound transducer 1 relative to the patient can be determined. This means that an approximate position of the 3D image dataset relative to the ultrasound transducer 1 can be derived. Only simulated sets in a limited search space around this approximate relative position might be selected for comparison with the measured set.

**Claims**

1. A data processing method, executed by a computer (11), of calculating a registration of a 3D image dataset of a patient taken at a first point in time with ultrasound data of the patient taken at a second point in time later than the first point in time, the method comprising the steps of:

   - acquiring (S04) the ultrasound data comprising a measured set of measured ultrasound A mode signals,
   - generating (S02) an ultrasound model including acoustic tissue properties from the 3D image dataset,
   - calculating (S03) a plurality of simulated sets of ultrasound A mode signals for different relative positions between the 3D image dataset and the ultrasound data by simulating the propagation of an ultrasound signal through the ultrasound model,
   - calculating (S05) the registration for which the similarity between the measured set and a simulated set of simulated ultrasound A mode signals generated from the 3D image dataset is maximized by using the simulated set out of the plurality of simulated sets for which the similarity is the largest.

2. The method of claim 1, wherein the simulated set of simulated ultrasound A mode signals is calculated for a virtual position of an ultrasound transducer used for measuring the measured ultrasound A mode signals relative to the 3D image data, wherein the ultrasound transducer has a plurality of ultrasound detectors and each ultrasound detector measures one of the measured ultrasound A mode signals in the measured set.

3. The method of any one of claims 1 or 2, wherein the similarity is maximized using an optimizer like Covariance Matrix Adaptation Evolution Strategy.

4. The method of any one of claims 1 to 3, wherein the similarity is optimized in a multi-dimensional search space.

5. The method of claim 4, wherein calculating the registration involves the steps of:

   - forming a thinned-out measured subset from the measured set,
   - forming a thinned-out simulated subset from the simulated set, the thinned-out simulated subset corresponding to the thinned-out measured subset,
   - calculating an intermediate registration for which the similarity between the thinned-out measured set and the thinned-out simulated set is maximized,
   - setting a limited search space around the intermediate registration, and
   - calculating the registration for which the similarity between the measured set and a simulated set is maximized within the limited search space.

6. The method of claim 4 or 5, wherein calculating the registration involves the steps of:

- tracking the position of the ultrasound transducer used for measuring the measured set relative to the patient,
- setting a limited search space based on the position of the ultrasound transducer, and
- calculating the registration for which the similarity between the measured set and a simulated set is maximized within the limited search space.

7. The method of claim 6, further comprising the steps of

- registering an atlas of the patient with the 3D image dataset, and
- determining the position of the 3D image dataset relative to the patient based on the registered atlas,

wherein the step of setting the limited search space is also based on the position of the 3D image dataset relative to the patient.

8. The method of any one of claims 4 to 7, wherein calculating the registration involves the steps of:

- registering an atlas of the patient with the 3D image dataset,
- determining the surface of the patient from the registered atlas, and
- setting a limited search space based on the surface of the patient.

9. The method of any one of claims 1 to 8, wherein

- the simulated set is smaller than the measured set, wherein the simulated ultrasound A mode signals to be calculated are set based on marks in the 3D image dataset or labels in an atlas matched to the 3D image dataset, and
- the similarity is calculated from measured ultrasound A mode signals in the measured set corresponding to the simulated ultrasound A mode signals in the simulated set.

10. The method of any one of claims 1 to 9, wherein calculating the registration involves the steps of:

- grouping simulated ultrasound A mode signals from the simulated set into at least one simulated group,
- calculating a simulated group signal for each of the at least one simulated group,
- grouping measured ultrasound A mode signals from the measured set into at least one measured group corresponding to the at least one simulated group, and
- calculating a measured group signal for each of the measured groups, wherein

the similarity in the step of calculating the registration is the similarity between the simulated group signals and the measured group signals.

11. A computer program which, when running on a computer (11), causes the computer (11) to perform the steps of any one of claims 1 to 10.

12. A computer (11) comprising a processor configured to perform the steps of any of claims 1 to 10.

13. A non-transitory computer readable storage medium on which the program according to claim 11 is stored.

**Patentansprüche**

1. Ein von einem Computer (11) ausgeführtes Datenverarbeitungsverfahren zum Berechnen einer Registrierung eines 3D-Bilddatensatzes eines Patienten, der zu einem ersten Zeitpunkt aufgenommen wurde, mit Ultraschalldaten des Patienten, die zu einem zweiten Zeitpunkt aufgenommen wurden, der später als der erste Zeitpunkt ist, wobei das Verfahren die folgenden Schritte umfasst:

- das Erfassen (S04) der Ultraschalldaten, die einen gemessenen Satz von gemessenen Ultraschall-A-Modus-Signalen umfassen,

- das Erzeugen (S02) eines Ultraschallmodells, das akustische Gewebeeigenschaften aus dem 3D-Bilddatensatz enthält,

- das Berechnen (S03) mehrerer simulierter Sätze von Ultraschall-A-Modus-Signalen für verschiedene relative Positionen zwischen dem 3D-Bilddatensatz und den Ultraschalldaten, durch Simulieren der Ausbreitung eines Ultraschallsignals durch das Ultraschallmodell,

- das Berechnen (S05) der Registrierung, für die die Ähnlichkeit zwischen dem gemessenen Satz und einem simulierten Satz von simulierten Ultraschall-A-Modus-Signalen, die aus dem 3D-Bilddatensatz erzeugt wurden, maximiert wird, indem der simulierte Satz aus mehreren simulierten Sätzen verwendet wird, für den die Ähnlichkeit am größten ist.

2. Das Verfahren nach Anspruch 1, wobei der simulierte Satz von simulierten Ultraschall-A-Modus-Signalen für eine virtuelle Position eines Ultraschallwandlers, der zum Messen der gemessenen Ultraschall-A-Modus-Signale verwendet wird, relativ zu den 3D-Bilddaten berechnet wird, wobei der Ultraschallwandler mehrere Ultraschalldetektoren aufweist und jeder Ultraschalldetektor eines der gemessenen Ultraschall-A-Modus-Signale im gemessenen Satz misst.

3. Das Verfahren nach einem der Ansprüche 1 oder 2, wobei die Ähnlichkeit unter Verwendung eines Optimierers wie der Covariance Matrix Adaptation Evolution Strategy maximiert wird.

4. Das Verfahren nach einem der Ansprüche 1 bis 3, wobei die Ähnlichkeit in einem mehrdimensionalen Suchraum optimiert wird.

5. Das Verfahren nach Anspruch 4, wobei das Berechnen der Registrierung die folgenden Schritte umfasst:

- das Bilden einer ausgedünnten gemessenen Teilmenge aus der gemessenen Menge,
- das Bilden einer ausgedünnten simulierten Teilmenge aus der simulierten Menge, wobei die ausgedünnte simulierte Teilmenge der ausgedünnten gemessenen Teilmenge entspricht,
- das Berechnen einer Zwischenregistrierung, für die die Ähnlichkeit zwischen der ausgedünnten gemessenen Menge und der ausgedünnten simulierten Menge maximiert ist,
- das Festlegen eines begrenzten Suchraums um die Zwischenregistrierung herum und
- das Berechnen der Registrierung, für die die Ähnlichkeit zwischen der gemessenen Menge und einer simulierten Menge innerhalb des begrenzten Suchraums maximiert ist.

6. Das Verfahren nach Anspruch 4 oder 5, wobei das Berechnen der Registrierung die folgenden Schritte umfasst:

- das Verfolgen der Position des Ultraschallwandlers, der zum Messen der gemessenen Menge verwendet wird, relativ zum Patienten,
- das Festlegen eines begrenzten Suchraums basierend auf der Position des Ultraschallwandlers und
- das Berechnen der Registrierung, für die die Ähnlichkeit zwischen der gemessenen Menge und einer simulierten Menge innerhalb des begrenzten Suchraums maximiert wird.

7. Das Verfahren nach Anspruch 6, das ferner die folgenden Schritte umfasst:

- das Registrieren eines Atlas des Patienten mit dem 3D-Bilddatensatz und
- das Bestimmen der Position des 3D-Bilddatensatzes relativ zum Patienten auf der Grundlage des registrierten Atlas,

wobei der Schritt des Festlegens des begrenzten Suchraums ebenfalls auf der Position des 3D-Bilddatensatzes relativ zum Patienten basiert.

8. Das Verfahren nach einem der Ansprüche 4 bis 7, wobei das Berechnen der Registrierung die folgenden Schritte umfasst:

- das Registrieren eines Atlas des Patienten mit dem 3D-Bilddatensatz,
- das Bestimmen der Oberfläche des Patienten aus dem registrierten Atlas und
- das Einrichten eines begrenzten Suchraums auf der Grundlage der Oberfläche des Patienten.

**9.** Das Verfahren nach einem der Ansprüche 1 bis 8, wobei

- der simulierte Satz kleiner ist als der gemessene Satz, wobei die zu berechnenden simulierten Ultraschall-A-Modus-Signale auf der Grundlage von Markierungen im 3D-Bilddatensatz oder Beschriftungen in einem Atlas, der dem 3D-Bilddatensatz zugeordnet ist, festgelegt werden, und
- die Ähnlichkeit aus gemessenen Ultraschall-A-Modus-Signalen im gemessenen Satz berechnet wird, die den simulierten Ultraschall-A-Modus-Signalen im simulierten Satz entsprechen.

**10.** Das Verfahren nach einem der Ansprüche 1 bis 9, wobei das Berechnen der Registrierung die folgenden Schritte umfasst:

- das Gruppieren simulierter Ultraschall-A-Modus-Signale aus dem simulierten Satz in mindestens eine simulierte Gruppe,
- das Berechnen eines simulierten Gruppensignals für jede der mindestens einen simulierten Gruppe,
- das Gruppieren gemessener Ultraschall-A-Modus-Signale aus dem gemessenen Satz in mindestens eine gemessene Gruppe, die der mindestens einen simulierten Gruppe entspricht, und
- das Berechnen eines gemessenen Gruppensignals für jede der gemessenen Gruppen, wobei

die Ähnlichkeit im Schritt des Berechnens der Registrierung die Ähnlichkeit zwischen den simulierten Gruppensignalen und den gemessenen Gruppensignalen ist.

**11.** Ein Computerprogramm, das, wenn es auf einem Computer (11) ausgeführt wird, den Computer (11) veranlasst, die Schritte eines der Ansprüche 1 bis 10 auszuführen.

**12.** Ein Computer (11) mit einem Prozessor, der dazu eingerichtet ist, die Schritte eines der Ansprüche 1 bis 10 auszuführen.

**13.** Ein nicht-flüchtiges, computerlesbares Speichermedium, auf dem das Programm gemäß Anspruch 11 gespeichert ist.

## Revendications

**1.** Procédé de traitement de données, exécuté par un ordinateur (11), pour calculer un recalage d'un ensemble de données d'images 3D d'un patient prises à un premier moment sur des données échographiques du patient prises à un deuxième moment postérieur au premier moment, le procédé comprenant les étapes suivantes :

- l'acquisition (S04) des données échographiques comprenant un ensemble mesuré de signaux échographiques en mode A mesurés,
- la génération (S02) d'un modèle échographique comprenant des propriétés acoustiques des tissus à partir de l'ensemble de données d'images 3D,
- le calcul (S03) d'une pluralité d'ensembles simulés de signaux échographiques en mode A pour différentes positions relatives entre l'ensemble de données d'images 3D et les données échographiques par simulation de la propagation d'un signal échographique à travers le modèle échographique,
- le calcul (S05) du recalage pour lequel la similarité entre l'ensemble mesuré et un ensemble simulé de signaux échographiques en mode A généré à partir de l'ensemble de données d'images 3D est maximisée, au moyen de celui desdits ensembles simulés pour lequel la similarité est la plus grande.

**2.** Procédé selon la revendication 1, dans lequel l'ensemble simulé de signaux échographiques en mode A simulés est calculé pour une position virtuelle d'un transducteur échographique servant à mesurer les signaux échographiques en mode A mesurés par rapport aux données d'images 3D, le transducteur échographique présentant une pluralité de détecteurs échographiques et chaque détecteur échographique mesurant un des signaux échographiques en mode A mesurés de l'ensemble mesuré.

**3.** Procédé selon l'une quelconque des revendications 1 et 2, dans lequel la similarité est maximisée à l'aide d'un optimiseur tel que CMA-ES (covariance matrix adaptation evolution strategy).

**4.** Procédé selon l'une quelconque des revendications 1 à 3, dans lequel la similarité est optimisée dans un espace de recherche multidimensionnel.

5. Procédé selon la revendication 4, dans lequel le calcul du recalage demande les étapes suivantes :

- la formation d'un sous-ensemble mesuré aminci à partir de l'ensemble mesuré,
- la formation d'un sous-ensemble simulé aminci à partir de l'ensemble simulé, le sous-ensemble simulé aminci correspondant au sous-ensemble mesuré aminci,
- le calcul d'un recalage intermédiaire pour lequel la similarité entre l'ensemble mesuré aminci et l'ensemble simulé aminci est maximisée,
- la définition d'un espace de recherche limité autour du recalage intermédiaire, et
- le calcul du recalage pour lequel la similarité entre l'ensemble mesuré et un ensemble simulé est maximisée au sein de l'espace de recherche limité.

6. Procédé selon la revendication 4 ou 5, dans lequel le calcul du recalage demande les étapes suivantes :

- le suivi de la position du transducteur échographique servant à mesurer l'ensemble mesuré par rapport au patient,
- la définition d'un espace de recherche limité en fonction de la position du transducteur échographique, et
- le calcul du recalage pour lequel la similarité entre l'ensemble mesuré et un ensemble simulé est maximisée au sein de l'espace de recherche limité.

7. Procédé selon la revendication 6, comprenant en outre les étapes suivantes :

- le recalage d'un atlas du patient sur l'ensemble de données d'images 3D, et
- la détermination de la position de l'ensemble de données d'images 3D par rapport au patient en fonction de l'atlas recalé ;

l'étape de définition de l'espace de recherche limité se faisant également en fonction de la position de l'ensemble de données d'images 3D par rapport au patient.

8. Procédé selon l'une quelconque des revendications 4 à 7, dans lequel le calcul du recalage demande les étapes suivantes :

- le recalage d'un atlas du patient sur l'ensemble de données d'images 3D,
- la détermination de la surface du patient à partir de l'atlas recalé, et
- la définition d'un espace de recherche limité en fonction de la surface du patient.

9. Procédé selon l'une quelconque des revendications 1 à 8, dans lequel

- l'ensemble simulé est plus petit que l'ensemble mesuré, les signaux échographiques en mode A simulés à calculer étant définis en fonction de repères présents dans l'ensemble de données d'images 3D ou d'étiquettes présentes dans un atlas correspondant à l'ensemble de données d'images 3D, et
- la similarité est calculée à partir des signaux échographiques en mode A mesurés de l'ensemble mesuré qui correspondent aux signaux échographiques en mode A simulés de l'ensemble simulé.

10. Procédé selon l'une quelconque des revendications 1 à 9, dans lequel le calcul du recalage demande les étapes suivantes :

- le regroupement des signaux échographiques en mode A simulés issus de l'ensemble simulé en au moins un groupe simulé,
- le calcul d'un signal de groupe simulé pour le ou les groupes simulés,
- le regroupement des signaux échographiques en mode A mesurés issus de l'ensemble mesuré en au moins un groupe mesuré correspondant à l'au moins un groupe simulé, et
- le calcul d'un signal de groupe mesuré pour chacun des groupes mesurés ; étant entendu que la similarité, dans l'étape de calcul du recalage, est une similarité entre les signaux de groupe simulé et les signaux de groupe mesuré.

11. Programme informatique qui, lorsqu'il est exécuté sur un ordinateur (11), amène l'ordinateur (11) à effectuer les étapes de l'une quelconque des revendications 1 à 10.

12. Ordinateur (11) comprenant un processeur conçu pour réaliser les étapes de l'une quelconque des revendications 1 à 10.

13. Support de stockage non transitoire lisible par ordinateur sur lequel est stocké le programme selon la revendication 11.

**Figure 1**

**Figure 2**

**Figure 3**

**Figure 4**

acquire 3D image
dataset
S01

generate US
model
S02

generate simu-
lated US data
S03

acquire measured
US data
S04

calculate
registration
S05

# Figure 5

**Figure 6**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6106464 A

**Non-patent literature cited in the description**

- User-interactive registration of bone with a-mode ultrasound. **HEGER S et al.** IEEE ENGINEERING IN MEDICINE AND BIOLOGY MAGAZINE. IEEE SERVICE CENTER, 01 March 2005, vol. 24, 85-95
- **AL-HASSAN ALY et al.** On Ultrasound Imaging for Guided Screw Insertion in Spinal Fusion Surgery. *ULTRASOUND IN MEDICINE AND BIOLOGY*, 14 January 2011, vol. 37 (4), 651-664
- **J.A. JENSEN**. Field: A Program for Simulating Ultrasound Systems, Paper. *10th Nordic-Baltic Conference on Biomedical Imaging Published in Medical & Biological Engineering & Computing*, 1996, vol. 34 (1), 351-353
- **J.A. JENSEN** ; **N. B. SVENDSEN**. Calculation of pressure fields from arbitrarily shaped, apodized, and excited ultrasound transducers. *IEEE Trans. Ultrason., Ferroelec., Freq. Contr.*, 1992, vol. 39, 262-267
- **B. E. TREEBY** ; **B. T. COX**. k-Wave: MATLAB toolbox for the simulation and reconstruction of photoacoustic wave-fields. *J. Biomed. Opt.*, 2010, vol. 15 (2), 021314
- **GARCIA, DAMIEN**. SIMUS: an open-source simulator for ultrasound imaging. Part I: theory & examples. *arXiv preprint arXiv:2102.02738*, 2021
- **CIGIER** ; **AMANDA** ; **FRANÇOIS VARRAY** ; **DAMIEN GARCIA**. SIMUS: an open-source simulator for ultrasound imaging. Part II: comparison with three popular simulators. *arXiv preprint arXiv:2103.05521*, 2021

- **DICKEY, FRED M.** ; **LOUIS A. ROMERO.** Normalized correlation for pattern recognition. *Optics letters*, 1991, vol. 16 (15), 1186-1188
- Mutual information analysis. **GIERLICHS** ; **BENEDIKT et al.** International Workshop on Cryptographic Hardware and Embedded Systems. Springer, 2008
- **BECKE, AXEL D**. Density-functional thermochemistry. II. The effect of the Perdew-Wang generalized-gradient correlation correction. *The Journal of chemical physics*, 1992, vol. 97 (12), 9173-9177
- **SUN, CHANGMING**. Symmetry detection using gradient information. *Pattern Recognition Letters*, 1995, vol. 16 (9), 987-996
- CMA-ES with restarts for solving CEC 2013 benchmark problems. **LOSHCHILOV, ILYA**. 2013 IEEE Congress on Evolutionary Computation.. Ieee, 2013
- **DILLENSEGER** ; **JEAN-LOUIS** ; **LAGUITTON** ; **SOIZIC** ; **DELABROUSSE** ; **ERIC**. Fast simulation of ultrasound images from a CT volume. *Computers in biology and medicine*, 2009, vol. 39, 180-6
- Real-Time Simulation of Medical Ultrasound from CT Images. **SHAMS R** ; **HARTLEY R** ; **NAVAB N.** Medical Image Computing and Computer-Assisted Intervention - MICCAI 2008. MICCAI 2008. Lecture Notes in Computer Science. Springer, 2008, vol. 5242
- **WEIN W.** ; **KHAMENE A** ; **CLEVERT DA** ; **KUTTER O.** ; **NAVAB N.** Simulation and Fully Automatic Multimodal Registration of Medical Ultrasound. *In: Ayache N., Ourselin S., Maeder*, 2007